Europäisches Patentamt

European Patent Office

Office européen des brevets

(19)

(11) Veröffentlichungsnummer : **0 468 290 B1**

(12) **EUROPÄISCHE PATENTSCHRIFT**

(45) Veröffentlichungstag der Patentschrift :
**02.03.94 Patentblatt 94/09**

(51) Int. Cl.⁵ : **C07C 45/35,** C07C 47/22, B01J 23/88, B01J 27/192

(21) Anmeldenummer : **91111542.6**

(22) Anmeldetag : **11.07.91**

(54) **Verfahren zur katalytischen Gasphasenoxidation von Propen oder iso-Buten zu Acrolein oder Methacrolein.**

(30) Priorität : **21.07.90 DE 4023239**

(43) Veröffentlichungstag der Anmeldung :
**29.01.92 Patentblatt 92/05**

(45) Bekanntmachung des Hinweises auf die
Patenterteilung :
**02.03.94 Patentblatt 94/09**

(84) Benannte Vertragsstaaten :
**BE DE FR GB IT**

(56) Entgegenhaltungen :
**DE-A- 2 611 249**

(73) Patentinhaber : **BASF Aktiengesellschaft
Carl-Bosch-Strasse 38
D-67063 Ludwigshafen (DE)**

(72) Erfinder : **Martan, Hans, Dr.
Mozartstrasse 62
W-6710 Frankenthal (DE)**
Erfinder : **Wegerle, Ulrike
St.-Bonifatius-Strasse 11
W-6520 Worms 28 (DE)**
Erfinder : **Ruppel, Wilhelm, Dr.
Koenigsberger Strasse 13
W-6830 Schwetzingen (DE)**
Erfinder : **Riekert, Lothar, Prof. Dr.
Im Eichbaeumle 21
W-7500 Karlsruhe (DE)**
Erfinder : **Becker, Dieter
Bad Sodener Strasse 2
W-6231 Sulzbach a. T. (DE)**
Erfinder : **Kotter, Michael, Dr.
Fruehmessweinberg 22
W-7520 Bruchsal (DE)**

## Beschreibung

Die vorliegende Erfindung betrifft ein neues Verfahren zur katalytischen Gasphasenoxidation von Propen oder iso-Buten zu Acrolein oder Methacrolein in einem Kontaktrohr-Festbettreaktor bei erhöhter Temperatur an katalytisch aktiven Oxiden mit einem Umsatz des Ausgangsolefins bei einfachem Durchgang von $\geq$90 %.

Die Gasphasenoxidation von Propen oder iso-Buten zu Acrolein oder Methacrolein ist insbesondere als erste Oxidationsstufe bei der Herstellung von Acryl- oder Methacrylsäure durch zweistufige katalytische Gasphasenoxidation von Propen oder iso-Buten in zwei hintereinandergeschalteten Reaktionsstufen von Bedeutung.

Sie verläuft stark exotherm, weshalb es infolge einer Vielfalt von möglichen Parallel- oder Folgereaktionen im Hinblick auf eine möglichst selektive Umsetzung von Propen zu Acrolein sowie von iso-Buten zu Methacrolein notwendig ist, den Verlauf der Reaktionstemperatur zu steuern.

Aus der DE-C 25 13 405 ist bekannt, bei Propenumsätzen bei einfachem Durchgang von mindestens 90 % den Verlauf der Reaktionstemperatur der katalytischen Gasphasenoxidation von Propen zu Acrolein in einem Vielrohr-Festbettreaktor an katalytisch aktiven Oxiden so zu steuern, daß durch den die Kontaktrohre umgebenden Raum eine 330°C aufweisende Salzschmelze zirkuliert und das die Reaktanden enthaltende Reaktionsgemisch der Reaktionszone bereits auf 330°C vorgeheizt zugeführt wird. In der DE-AS 20 56 614 sowie der DE-A 30 06 894 wird darüberhinaus auf den Verlauf der Reaktionstemperatur der katalytischen Gasphasenoxidation von Propen zu Acrolein dadurch Einfluß genommen, daß die Aktivität der Katalysatormasse durch Verdünnen mit inertem Material beziehungsweise durch Variation der Zusammensetzung in Strömungsrichtung zunimmt. Nachteilig an diesen Verfahren ist jedoch, daß die so implizit längs der Kontaktrohre eingestellten Verläufe der Reaktionstemperatur im Hinblick auf eine möglichst selektive Umsetzung des Ausgangsolefins zum Aldehyd nicht voll zu befriedigen vermögen.

Aufgabe der vorliegenden Erfindung war daher, ein Verfahren zur katalytischen Gasphasenoxidation von Propen oder iso-Buten zu Acrolein oder Methacrolein in einem Kontaktrohr-Festbettreaktor bei erhöhter Temperatur an katalytisch aktiven Oxiden mit einem Umsatz des Ausgangsolefins bei einfachem Durchgang von $\geq$90 % zur Verfügung zu stellen, das einen im Hinblick auf eine erhöhte Selektivität der Aldehydbildung verbesserten Verlauf der Reaktionstemperatur aufweist.

Demgemäß wurde ein Verfahren zur katalytischen Gasphasenoxidation von Propen oder iso-Buten zu Acrolein oder Methacrolein in einem Kontaktrohr-Festbettreaktor bis erhöhter Temperatur an katalytisch aktiven Oxiden mit einem Umsatz des Ausgangsolefins bei einfachem Durchgang von $\geq$90 % gefunden, das dadurch gekennzeichnet ist, daß die Reaktionstemperatur in Strömungsrichtung längs der Kontaktrohre ab Eintritt der die Reaktanden enthaltenden Reaktionsgase in die Kontaktrohre bis zum Erreichen eines Umsatzes des Ausgangsolefins von 30 bis 70 % 360 bis 420°C beträgt, daran anschließend bis zum Erreichen eines Umsatzes des Ausgangsolefins von 80 bis 90 % auf 360 bis 300°C eingestellt und danach bis zum Austritt der Reaktionsgase aus den Kontaktrohren bei 330 bis 390°C gehalten wird.

Als oxidische Katalysatoren eignen sich insbesondere Massen der allgemeinen Formel I

$$Mo_{12}Bi_aFe_bX^1_cX^2_dX^3_eX^4_fO_n \qquad (I),$$

in der die Variablen folgende Bedeutung haben:

$X^1$      Nickel und/oder Kobalt,
$X^2$      Thallium, ein Alkalimetall und/oder ein Erdalkalimetall,
$X^3$      Phosphor, Arsen, Bor, Antimon, Zinn, Cer, Blei, Niob und/oder Wolfram,
$X^4$      Silicium, Aluminium, Titan und/oder Zirkonium,
a      0,5 bis 5,0 ,
b      0,01 bis 3,0 ,
c      3,0 bis 10,0 ,
d      0,02 bis 2,0 ,
e      0 bis 5,0 ,
f      0 bis 10 und
n      eine Zahl, die durch die Wertigkeit und Häufigkeit der von Sauerstoff verschiedenen Elemente in I bestimmt wird.

Besonders bevorzugt sind Massen der Formel
$Mo_{12}Bi_1Fe_2Ni_{8,5}P_{0,06}Na_{0,18}K_{0,06}Si_{10}O_n$ (für die Oxidation von Propen zu Acrolein) sowie der Formel $Mo_{12}Bi_1Fe_3Ni_1Co_7B_2Sb_{0,1}K_{0,14}O_n$ (für die Oxidation von iso-Buten zu Methacrolein).

Die Massen I sind in an sich bekannter Weise erhältlich. Sie können beispielsweise dadurch hergestellt werden, daß man als Ausgangsverbindungen geeignete Salze der sie konstituierenden elementaren Bestandteile, gegebenenfalls bei erhöhter Temperatur und unter Zusatz von Säuren oder Basen, in wäßrigem Medium durch Lösen und/oder Suspendieren fein verteilt, mischt, das Gemisch zur Trockene eindampft, die erhaltene Masse formt und in der Regel bei Temperaturen von 180 bis 480°C, vorzugsweise 350 bis 450°C im Luftstrom oder in inerter Atmosphäre, z.B. $N_2$ oder $CO_2$, calciniert. Bei der Formung können an sich bekannte Hilfsmittel wie Gleitmittel (z.B. Graphit) oder Formhilfsmittel und Verstärkungsmittel wie Mikrofasern aus Glas, Asbest, Siliciumcarbid oder Kaliumtitanat zugesetzt werden. In dieser Form werden die oxidischen Massen I zweckmäßigerweise zur Verwendung als Vollkatalysatoren hergestellt, wobei Hohlzylinder mit einem Außendurchmesser und einer Länge von 4 bis 10 mm und einer Wandstärke von 1 bis 3 mm die bevorzugte Katalysatorgeometrie darstellen. Die katalytisch aktiven Oxide können aber auch in Gestalt von Schalenkatalysatoren, das heißt auf ein vorgeformtes Trägermaterial aufgebracht, angewendet werden, wobei das Aufbringen auf das Trägermaterial z.B. in Form einer wäßrigen Ausgangslösung oder Suspension, verbunden mit anschließender Trocknung und Calcinierung, oder als bereits calcinierte pulverisierte Masse in Kombination mit einem Bindemittel erfolgen kann.

Selbstverständlich können die katalytisch aktiven oxidischen Massen auch in Pulverform als Katalysatoren eingesetzt werden.

Das Oxidationsmittel Sauerstoff kann z.B. in Form von Luft, aber auch in reiner Form eingesetzt werden. Vorzugsweise werden die Reaktionspartner mit Inertgas wie $N_2$, rückgeführten Reaktionsabgasen und/oder Wasserdampf verdünnt. In der Regel wird bei einem Ausgangsolefin:Sauerstoff:indifferente Gase (einschließlich Wasserdampf) Volumen(N1)-Verhältnis von 1:(1,0 bis 3,0): (5 bis 25), vorzugsweise 1:(1,7 bis 2,3):(10 bis 15) gearbeitet. Der Reaktionsdruck liegt überlicherweise im Bereich von 1 bis 3 bar und die Gesamtraumbelastung beträgt vorzugsweise 1500 bis 2500 Nl/l/h. Bei dem beschriebenen Verfahren wird kein reines Acrolein oder Methacrolein, sondern ein Gasgemisch erhalten, von dessen Nebenkomponenten das Acrolein oder Methacrolein in an sich bekannter Weise abgetrennt werden kann. Bei einer Verwendung des Acroleins oder Methacroleins zur Herstellung von Acryl- oder Methacrylsäure durch zweistufige katalytische Gasphasenoxidation von Propen oder iso-Buten werden die Acrolein oder Methacrolein enthaltenden Reaktionsgase in der Regel ohne Abtrennung der Nebenkomponenten in die zweite Oxidationsstufe überführt.

Die Realisierung des erfindungsgemäßen Profils der Reaktionstemperatur kann in an sich bekannter Weise erfolgen, z.B. durch abschnittsweise Heizung oder Kühlung des Kontaktrohres mittels elektrischer Temperierbänder oder zirkulierenden fluiden Temperiermedien wie Schmelzen von Salzen wie Kaliumnitrat, Natriumnitrit und/oder Natriumnitrat, oder von niedrig schmelzenden Metallen wie Natrium, Quecksilber sowie Legierungen verschiedener Metalle, wobei im Falle eines Einzelrohres infolge des hohen Wärmeübergangs die während der Reaktion im Rohrinneren herrschende Temperatur im wesentlichen gleich der äußeren Heiz- oder Kühltemperatur ist.

Abschnittsweises Heizen oder Kühlen ist aber auch bei Vielrohr-Festbettreaktoren möglich und z.B. in den Schriften DE-A 28 30 765, DE-A 22 01 528, DE-A 16 01 162, DE-A 25 13 405 sowie US-A 3 147 084 beschrieben. Eine weitere Möglichkeit der Steuerung der Reaktionstemperatur besteht im abschnittsweisen Erhöhen oder Senken der Katalysatoraktivität. Dies kann durch chemische Modifikation der aktiven Katalysatormasse, aber auch durch Verdünnen mit desaktiviertem Katalysator oder Inertmaterial realisiert werden (DE-A 20 56 614 und DE-A 20 06 894).

Gegebenenfalls kann auch abschnittsweises Heizen/Kühlen mit abschnittsweisem Erhöhen/Senken der Katalysatoraktivität kombiniert werden. Vorzugsweise werden die Ausgangsreaktionsgase in allen Fällen der ersten Reaktionszone bereits auf die entsprechende Reaktionstemperatur vorgeheizt zugeführt.

Typische Kontaktrohre bestehen aus V2A Stahl einer Wanddicke von etwa 2 mm und eines Innendurchmessers von 25 mm. Die Anzahl solcher Kontaktrohre in einem Vielrohr-Festbettreaktor beläuft sich in der Regel auf 10000 bis 30000. Umsatz U und Selektivität S sind in dieser Schrift wie folgt definiert:

$$U = \frac{\text{Molzahl umgesetztes Propen}}{\text{Molzahl eingesetztes Propen}} \times 100$$

$$S = \frac{\text{Molzahl Propen umgesetzt zu Acrolein}}{\text{Molzahl Propen insgesamt umgesetzt}} \times 100$$

Beispiel B und Vergleichsbeispiel V

B: Ein mittels elektrischer Heizbänder abschnittsweise temperierbares Stahlrohr (V2A, 2 mm Wanddicke, 25 mm Innendurchmesser) wurde mit einem Schalenkatalysator gemäß Beispiel 4a) der EP-B 17 000 gefüllt (2,50 m Füllhöhe) und mit 1800 l/l/h einer auf 380°C vorgeheizten Gasmischung der Zusammensetzung

5 Vol.-% Propen
45 Vol.-% Luft und
50 Vol.-% Stickstoff

beschickt. Bis zu einem Propenumsatz von 65 % wurde die Reaktionstemperatur längs des Kontaktrohres auf 380°C gehalten, anschließend wurde die Reaktionstemperatur längs des Kontaktrohres bis zu einem Propenumsatz von 85 % auf 335°C abgesenkt und danach bis zum Verlassen des Kontaktrohres auf 340°C gehalten. Der Umsatz U an Propen bei einfachem Durchgang betrug 95 % bei einer Selektivität S der Acroleinbildung von 93 %.

V: Es wurde wie in B gearbeitet, die Gasmischung war jedoch auf 350°C vorgeheizt und die Reaktionstemperatur wurde längs des gesamten Kontaktrohres bei 350°C gehalten. Der Umsatz U an Propen bei einfachem Durchgang betrug ebenfalls 95 % bei einer Selektivität S der Acroleinbildung von 88 %.

## Patentansprüche

1. Verfahren zur katalytischen Gasphasenoxidation von Propen oder iso-Buten zu Acrolein oder Methacrolein in einem Kontaktrohr-Festbettreaktor bei erhöhter Temperatur an katalytisch aktiven Oxiden mit einem Umsatz des Ausgangsolefins bei einfachem Durchgang von $\geqq 90$ %, dadurch gekennzeichnet, daß die Reaktionstemperatur in Strömungsrichtung längs der Kontaktrohre ab Eintritt der die Reaktanden enthaltenden Reaktionsgase in die Kontaktrohre bis zum Erreichen eines Umsatzes des Ausgangsolefins von 30 bis 70 % 360 bis 420°C beträgt, daran anschließend bis zum Erreichen eines Umsatzes des Ausgangsolefins von 80 bis 90 % auf 360 bis 300°C eingestellt und danach bis zum Austritt der Reaktionsgase aus den Kontaktrohren bei 330 bis 390°C gehalten wird.

2. Verfahren nach Anspruch 1, dadurch gekennzeichnet, daß als oxidische Katalysatoren Massen der allgemeinen Formel

$$Mo_{12}Bi_aFe_bX^1_cX^2_dX^3_eX^4_fO_n \qquad (I),$$

in der die Variablen folgende Bedeutung haben:

| | |
|---|---|
| $X^1$ | Nickel und/oder Kobalt, |
| $X^2$ | Thallium, ein Alkalimetall und/oder ein Erdalkalimetall, |
| $X^3$ | Phosphor, Arsen, Bor, Antimon, Zinn, Cer, Blei, Niob und/oder Wolfram, |
| $X^4$ | Silicium, Aluminium, Titan und/oder Zirkonium, |
| a | 0,5 bis 5,0 , |
| b | 0,01 bis 3,0 , |
| c | 3,0 bis 10,0 , |
| d | 0,02 bis 2,0 , |
| e | 0 bis 5,0 , |
| f | 0 bis 10 und |
| n | eine Zahl, die durch die Wertigkeit und Häufigkeit der von Sauerstoff verschiedenen Elemente in I bestimmt wird, |

verwendet werden.

## Claims

1. A process for the catalytic gas-phase oxidation of propene or isobutene to acrolein or methacrolein in a catalyst tube fixed-bed reactor at elevated temperature over catalytically active oxides with a conversion of the starting olefin of $\geqq$ 90 % for a single pass, wherein the reaction temperature in the flow direction along the catalyst tubes from entry of the reaction gases containing the reactants into the catalyst tubes is from 360 to 420°C until a conversion of the starting olefin of from 30 to 70 % has been reached, is subsequently adjusted to from 360 to 300°C until a conversion of the starting olefin of from 80 to 90 % has been reached, and is then kept at from 330 to 390°C until the reaction gases leave the catalyst tubes.

2. A process as claimed in claim 1, wherein the oxidic catalysts are compositions of the formula

$$Mo_{12}Bi_aFe_bX^1_cX^2_dX^3_eX^4_fO_n \qquad (I),$$

where

| | |
|---|---|
| $X^1$ | is nickel and/or cobalt, |
| $X^2$ | is thallium, an alkali metal and/or an alkaline earth metal |
| $X^3$ | is phosphorus, arsenic, boron, antimony, tin, cerium, lead, niobium and/or tungsten, |
| $X^4$ | is silicon, aluminum, titanium and/or zirconium, |
| a | is from 0.5 to 5.0 |
| b | is from 0.01 to 3.0, |

c       is from 3.0 to 10.0,
d       is from 0.02 to 2.0,
e       is from 0 to 5.0,
f       s from 0 to 10 and
n       is a number determined by the valency and frequency in I of the elements other than oxygen.


**Revendications**

1.  Procédé d'oxydation catalytique en phase gazeuse du propène ou de l'isobutène en acroléine ou méthacroléine dans un réacteur à tube à contact-lit fixe et à température élevée sur des oxydes catalytiquement actifs, avec une conversion de l'oléfine de départ pour un passage unique égale ou supérieure à 90, caractérisé en ce que l'on règle la température réactionnelle dans la direction d'écoulement le long des tubes à contact depuis l'entrée des gaz de réaction contenant les réactifs dans tubes à contact jusqu'à l'obtention d'une conversion de l'oléfine de départ de 30 à 70%, à 360-420°C, puis jusqu'à l'obtention d'une conversion de l'oléfine de départ de 80 à 90%, à 360-300°C et on la maintient ensuite, jusqu'à la sortie des gaz de réaction des tubes à contact, à 330-390°C.

2.  Procédé suivant la revendication 1, caractérisé en ce que, à titre de catalyseurs oxydiques, on utilise des matières de la formule générale

$$Mo_{12}Bi_aFe_bX^1_cX^2_dX^3_eX^4_fO_n \qquad (I),$$

dans laquelle les diverses variables ont les significations suivantes :

$X^1$       nickel et/ou cobalt,
$X^2$       thallium, un métal alcalin et/ou un métal alcalino-terreux,
$X^3$       phosphore, arsenic, bore, antimoine, étain, cérium, plomb, niobium et/ou tungstène,
$X^4$       silicium, aluminium, titane et/ou zirconium
a       0,5 à 5,0
b       0,01 à 3,0
c       3,0 à 10,0
d       0,02 à 2,0
e       0 à 5,0
f       0 à 10 et
n       un nombre déterminé par la valence et la fréquence des divers éléments qui diffèrent de l'oxy-gène dans I.